# EUROPEAN PATENT SPECIFICATION

(11) **EP 0 696 439 B1**
(45) Date of publication and mention of the grant of the patent: **02.09.1998**
(21) Application number: 95305477.2
(22) Date of filing: 04.08.1995
(51) Int. Cl.: A61B 17/58, A61F 2/46

(54) **Acetabular bone graft impactor**
Werkzeug zum Verdichten eines acetabularen Knochentransplantats
Outil pour comprimer une greffe osseuse acétabulaire

(30) Priority: 11.08.1994 GB 9416215
(43) Date of publication of application: 14.02.1996
(73) Proprietor: HOWMEDICA INTERNATIONAL INC., Shannon Co. Clare (IE)
(72) Inventor: Ashby, Alan, F-14000 Caen (FR); Pichon, Denis, Shepperton, Middlesex, TW17 8BS (GB); Slooff, Tom Johanes, Prof. Dr., NL-5843 AB Westerbeek (NL)
(74) Representative: Bridge-Butler, Alan James

(56) References cited:
- EP-A- 0 357 270
- EP-A- 0 535 973
- WO-A-86/05384
- DE-U- 8 623 700
- FR-A- 2 233 972
- US-A- 4 904 265

## Description

This invention relates to an acetabular bone graft impactor. Such instruments are used to reconstruct the acetabular cavity with morselised bone graft in case of bone defects.

The technique of morselised bone grafting has been used for the construction of bone defects all around and in the acetabular cavity using simple basic instruments such as impactors. The shape of these impactors is usually hemispherical and used with a hammer in order to impact the bone graft in place of bone defects.

Impactors, as at present used, usually have a shape that is quasi similar to the shape of a healthy acetabulum. The most common defects are all around the acetabular cavity, known as the rim. Therefore, the strongest impaction is performed in the areas where there are no defects and no requirements for strong impaction. Due to a lack of bone support and perpendicular impaction to the opening of the cavity, the area where the strongest impaction is required is often very weakly or not impacted. This can lead to a failure of the surgical act, such as migration of the prosthetic component, resorbtion of the graft.

When performing the operation it is also possible to see the graft issuing out of the cavity during the impaction. This induces a waste of expensive bone graft, particularly in the area of the opening of the defect.

DE-U-86 23 700 shows an acetabular bone grafter having an expandible contact surface provided on an adjustable head. The contact surface is formed on a ring of plates which can be moved radially apart to increase the overall diameter. When the diameter is increased however the contact surface provided by the outer surface of the ring of plates is interrupted and becomes non-continuous.

The present invention is intended to provide the surgeon with an acetabular impactor that will avoid the above-mentioned problems by giving the surgeon the opportunity to impact the graft in the area of the defect he wants to fill As the acetabular in defects can have various shapes, the impactor is adaptable as required.

According to the present invention an acetabular bone graft impactor comprises a substantially rigid contact surface at least part of which is located on a head connected to a shaft provided with an anvil, characterised by means for altering the shape of the contact surface which include a releasable flange which when connected to the shaft and/or the head, projects radially outwardly beyond the contact surface on the head.

The flange is preferably a sector of a circle.

In an alternative construction according to the present invention the bone graft impactor comprises a substantially rigid contact surface at least part of which is located on a head connected by a shaft provided with an anvil characterised by including a second shaft provided with an anvil and to which the means for altering the shape and area of the contact surface is attached, said second shaft having means to receive the head of a first shaft and to which the head can be transferred, and said means for altering the shape of the contact surface including a flange attached to said second shaft and which projects radially outwardly beyond the contact surface on the head when in position on the second shaft.

The invention can be performed in numerous ways and three embodiments will now be described by way of example and with reference to the accompanying drawings in which :
Figure 1 is an exploded isometric view of the components of the acetabular bone graft impactor according to the invention;
Figure 2 is an isometric view of the impact components shown in Figure 1 in their assembled positions;
Figure 3 is a plan view of the attachable means for altering the shape of the contact surface of the construction shown in Figures 1 and 2;
Figure 4 is a plan view of a locking nut used in the construction shown in Figures 1 and 2;
Figure 5 is a plan view of an alternative construction of locking nut;
Figure 6 is an isometric view of the locking nut shown in Figure 5;
Figure 7 is an exploded isometric view of a further alternative construction according to the invention;

As shown in the drawings the acetabular bone graft impactor according to the invention comprises a metal or hard material head 1 which is of hemi-spherical shape and which has a contact surface 2 and a rear face 3. The head 1 is carried on a shaft 4 the other end of which is provided with an anvil 5. Thus, the impactor so far described is generally of known form.

In order to provide attachable means for altering the shape of the contact surface 2 the shaft 4 is provided with a screw thread 5 at its end adjacent the rear face of the head. The screw thread 5 is adapted to take a nut 6 which has a knurled rim 7. The nut is also provided with a slot 8 leading from its outer rim 7 to a screw threaded opening 9.

The attachable means itself is provided by a flange element 10 which has an opening 11 which leads to a slot 12 in an extension 13. The reminder of the element 10 is formed by a flange 14 in the form of a sector of a circle and in which is a location opening 15.

The rear face 3 of the head 1 is provided with a location boss 16 and the shaft 4 carries flats 17 the width across which is slightly less than the width across the faces of the slot 8 in the nut 6 and the slot 12 in the flange element 10.

In order to assemble the attachable means the flange element 10 is located on the shaft 4 by sliding the slot 12 over the flats 17 and then moving the element down the shaft until the opening 11 surround the screw thread 5. It will be appreciated that due to the keyhole shape of the opening 11 and slot 12 the element cannot be detached and it is located in a given angular position by engagement of the location opening 15 over the location boss 16 on the rear face 3 of the head 1.

The nut 6 is now placed in position in a similar manner and so that its screw threaded opening 19 can engage the screw thread 5 on the flange. Rotation of the nut can be sufficient to hold the flange element 10 firmly in position.

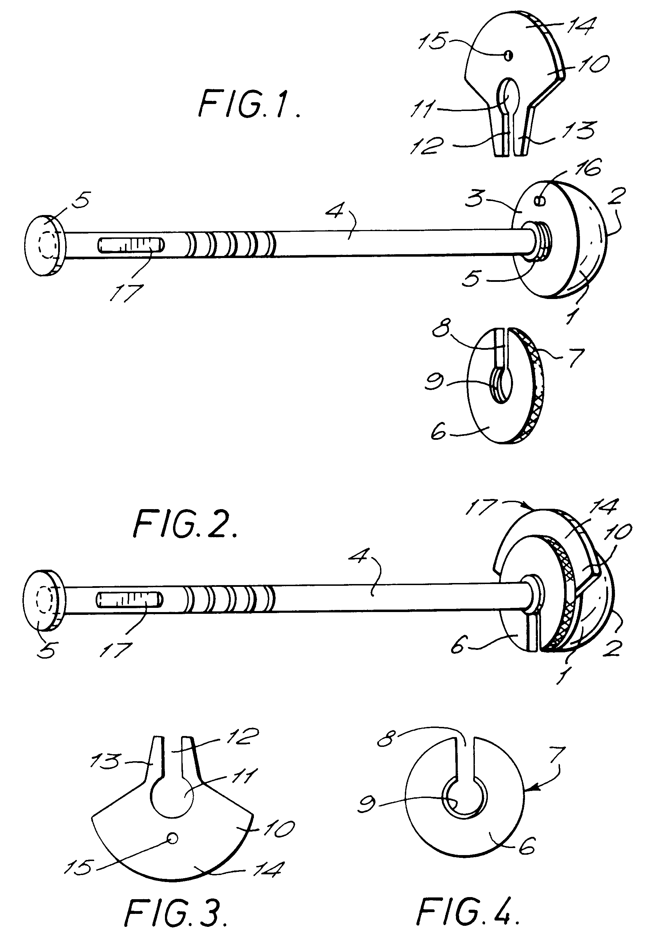

The assembled impactor is shown in Figure 2 with the flange 14 of the flange element 10 projecting radially outwardly from the head. The lower surface 17 of the flange 14 now acts to extend the contact surface 2 of the head 1 thus altering the shape of the total contact surface.

The flange element 10 can be made slightly flexible, for example by making it from a medical grade plastics material. Thus, the flange can be customised in the operating theatre using pliers or scissors. Being adaptable, once it has been customised, or not, it can be used with all sizes of impactors having, for example, different shaped heads 1 provided the shaft 4 or flats 17 and screw thread 5 are the same.

The shape of the flange fulfils two main requirements, the first for impacting the graft and the second for attachment to the impactors.

The impact ion of the graft is performed by the transmission of forces via the impactor to the flange and ultimately the graft. Thus the anvil 5 is struck appropriately by either an impact hammer or some other device.

It will be appreciated that the impactor can be used without the attachable means for altering the shape of the contact surface. Thus it can be used in the configuration shown in Figure 1 without the fittings. It is therefore possible to insert and disengage the attachable means, for example at the final stage of the fixing process.

The flange can also be made from a heavier material if desired and if so it can be pre-shaped as required.

The effect of the flange is to enable the outer perimeter of the acetabular socket to be compacted and prevent the problems referred to above.

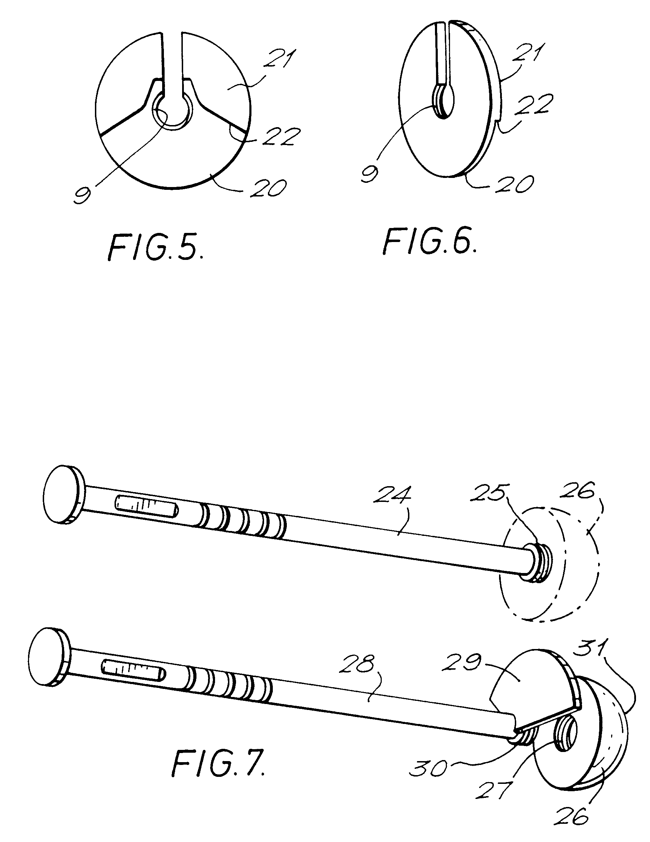

Figure 5 shows an alternative construction of locking nut 20 which can be used with a flange element 10 of the kind shown in Figure 3. In this construction the engaging face 21 of the lock nut 20 is cut away at 22 to form a shape similar to the shape of the outer perimeter of the engaging part of the flange element 10. As will be seen from Figure 6 the cut away 22 is of substantially the same depth as the thickness of the flange element 10 so that the appropriate portions of the flange element and the lock nut can be together. With this construction the location boss 16 on the rear face 3 of the head 1 is omitted but the locking but 20 enables the flange element 10 to be held firmly against the face 3 of the head when the lock nut is screwed down into position on the thread 5.

Figure 7 shows an alternative construction in which the shaft 24 is of similar construction to the shaft 4 described in the construction shown in Figure 1 but in which the end of the shaft carries a screw thread 25 to receive a hemispherical head 26 which is provided with a screw threaded bore 27 so that the head 26 can be screwed into the shaft 24. The head is shown in this position in chain lines at the upper part of the Figure .

A second shaft 28 is provided to which is rigidly connected a flange element 29 immediately above a screw-threaded end 30 the dimensions of which are similar to the screw threaded end 25 on the shaft 24.

When the first impaction has been completed the detachable head 26 can be removed from the stem 24 and attached by means of the screw thread 30 to the shaft 28, the flange element 10 thus acting to extend the contact surface 31 of the head 26 which therefore alters the shape of the total contact surface.

Once again the effect of the flange 29 is to enable the outer perimeter of the acetabular socket to be compacted to prevent the problem referred to above.

## Claims

1. An acetabular bone graft impactor comprising a substantially rigid contact surface (2) at least part of which is located on a head (1) connected to a shaft (4) provided with an anvil (5), characterised by means (14) for altering the shape of the contact surface (2) which include a releasable flange (14) which when connected to the shaft (4) and/or the head (5), projects radially outwardly beyond the contact surface on the head.

2. An acetabular bone graft impactor as claimed in claim 1 characterised in that said flange (14) is a sector of a circle.

3. An acetabular bone graft impactor comprising a substantially rigid contact surface (31) at least part of which is located on a head (26) connected by a shaft (24) provided with an anvil (5) characterised by including a second shaft (28) provided with an anvil (5) and to which means (29) for altering the shape and area of the contact surface is attached, said second shaft (28) having means (30) to receive the head (26) of a first shaft (24) and to which the head (26) can be transferred, and said means (29) for altering the shape of the contact surface including a flange (29) attached to said second shaft (28) and which projects radially outwardly beyond the contact surface on the head (26) when in position on the second shaft.

4. An acetabular bone graft impactor as claimed in claim 3 characterised in that said flange (29) is a sector of a circle.

## Patentansprüche

1. Knochentransplantatverdichter für Hüftgelenkpfannen, umfassend eine im wesentlichen starre Kontaktoberfläche (2), von der zumindest ein Teil auf einem Kopf (1) angeordnet ist, der mit einem Schaft (4) verbunden ist, welcher mit einem Amboß (5) versehen ist, gekennzeichnet durch eine Vorrichtung (14) zum Verändern der Form der Kontaktoberfläche (2), wobei die Vorrichtung einen abnehmbaren Flansch (14) einschließt, der über die Kontaktoberfläche des Kopfes hinaus radial nach außen übersteht, wenn er mit dem Schaft (4) und/oder dem Kopf (5) verbunden ist.

2. Knochentransplantatverdichter für Hüftgelenkpfannen nach Anspruch 1, dadurch gekennzeichnet, daß der Flansch (14) die Form eines Kreissektors aufweist.

3. Knochentransplantatverdichter für Hüftgelenkpfannen, umfassend eine im wesentlichen starre Kontaktoberfläche (31), von der zumindest ein Teil auf einem Kopf (26) angeordnet ist, der mit einen Schaft (24) verbunden ist, welcher mit einem Amboß (5) versehen ist, dadurch gekennzeichnet, daß ein zweiter Schaft (28) umfaßt ist, der mit einem Amboß (5) versehen ist und an dem eine Vorrichtung (29) zum Verändern der Form und Fläche der Kontaktoberfläche angebracht ist, wobei der zweite Schaft (28) eine Vorrichtung (30) zur Aufnahme des Kopfes (26) des ersten Schaftes (24) aufweist, auf welche der Kopf (26) verlagert werden kann, und die Vorrichtung (29) zum Verändern der Form der Kontaktoberfläche einen Flansch (29) einschließt, der an dem zweiten Schaft (28) angebracht ist und der über die Kontaktoberfläche auf dem Kopf (26) hinaus radial nach außen übersteht, wenn er sich auf dem zweiten Schaft in Position befindet.

4. Knochentransplantatverdichter für Hüftgelenkspfannen nach Anspruch 3, dadurch gekennzeichnet, daß der Flansch (29) die Form eines Kreissektors aufweist.

## Revendications

1. Impacteur de greffe osseuse acétabulaire comportant une surface de contact sensiblement rigide (2) dont au moins une partie est située sur une tête (1) reliée à un arbre (4) muni d'une enclume (5), caractérisé par des moyens (14) destinés à modifier la forme de la surface de contact (2) qui comportent une collerette détachable (14) qui, lorsqu'elle est reliée à l'arbre (4) et/ou à la tête (5), fait saillie radialement vers l'extérieur au-delà de la surface de contact de la tête.

2. Impacteur de greffe osseuse acétabulaire selon la revendication 1 caractérisé en ce que ladite collerette (14) est un secteur de cercle.

3. Impacteur de greffe osseuse acétabulaire comportant une surface de contact sensiblement rigide (31) dont au moins une partie est située sur une tête (26) reliée par un arbre (24) muni d'une enclume (5), caractérisé en ce qu'il comporte un second arbre (28) muni d'une enclume (5) et auquel sont fixés des moyens (29) destinés à modifier la forme et la surface de la surface de contact, ledit second arbre (28) ayant des moyens (30) pour recevoir la tête (26) du premier arbre (24) et vers lesquels la tête (26) peut être transférée, et lesdits moyens (29) destinés à modifier la forme de la surface de contact comportent une collerette (29) fixée audit second arbre (28) et qui fait saillie radialement vers l'extérieur au-delà de la surface de contact de la tête (26) lorsqu'elle est positionnée sur le second arbre.

4. Impacteur de greffe osseuse acétabulaire selon la revendication 3 caractérisé en ce que ladite collerette (29) est un secteur de cercle.
